# FASCICULE DE BREVET EUROPEEN

(11) **EP 2 235 195 B1**
(45) Date de publication et mention de la délivrance du brevet: **06.07.2011**
(21) Numéro de dépôt: 08872507.2
(22) Date de dépôt: 10.12.2008
(51) Int. Cl.: C12P 19/14, C12P 19/02, C12P 7/10

(54) **Procédé de production de jus sucré à partir de biomasse lignocellulosique avec recyclage amélioré des enzymes**
Verfahren zur Herstellung eines Zuckersafts aus Lignozellulose-haltiger Biomasse mit verbesserter Enzymrückgewinnung
Method for producing a sugar juice from a lignocellulosic biomass with improved enzyme recycling

(30) Priorité: 20.12.2007 FR 0709121
(43) Date de publication de la demande: 06.10.2010
(73) Titulaire: IFP Energies nouvelles, 92852 Rueil-Malmaison Cedex (FR)
(72) Inventeur: CASANAVE, Dominique, 69230 SAINT-GENIS LAVAL (FR); LOPEZ FERREIRA, Nicolas, F-28210 Croisilles (FR)
(86) Numéro de dépôt international: PCT/FR2008/001719
(87) Numéro de publication internationale: WO 2009/103878

(56) Documents cités:
- EP-A- 0 062 027
- JP-A- 2006 149 343
- US-A- 5 348 871
- US-A- 5 962 289
- DATABASE MEDLINE [Online] US NATIONAL LIBRARY OF MEDICINE (NLM), BETHESDA, MD, US; mars 2003 (2003-03), SHEN XUE-LIANG ET AL: "[Studies on immobilized cellobiase]" XP002498648 Database accession no. NLM15966329 & SHENG WU GONG CHENG XUE BAO = CHINESE JOURNAL OF BIOTECHNOLOGY MAR 2003, vol. 19, no. 2, mars 2003 (2003-03), pages 236-239, ISSN: 1000-3061
- SHEN XUELIANG ET AL: "Lactic acid production from cellulosic material by synergetic hydrolysis and fermentation." APPLIED BIOCHEMISTRY AND BIOTECHNOLOGY JUN 2006, vol. 133, no. 3, juin 2006 (2006-06), pages 251-262, XP002556173 ISSN: 0273-2289

## Description

### Domaine de l'invention

La présente invention s'inscrit dans la production de biocarburants dits de "seconde génération". Il concerne un procédé de production de jus sucrés dans lequel l'hydrolyse enzymatique de substrats lignocellulosiques présente une amélioration de l'utilisation et du recyclage des enzymes mises en jeu. Les jus sucrés ainsi obtenus sont envoyés vers l'étape de fermentation alcoolique puis de distillation, en vue de la production d'un alcool ex-biomasse.

### Étude de l'art antérieur

La ressource de biomasse lignocellulosique représente une source d'énergie renouvelable considérable et provient aussi bien des résidus agricoles et forestiers ou des sous-produits de transformation du bois que des cultures dédiées (plantes ligneuses ou herbacées). La matière lignocellulosique est constituée de trois éléments majeurs imbriqués dans un réseau complexe. Ces éléments sont la cellulose, les hémicelluloses et la lignine. Leurs proportions respectives varient selon l'origine exacte de la biomasse.

La voie souvent préconisée pour obtenir des sucres fermentescibles à partir de cellulose est l'hydrolyse enzymatique. La dégradation de la cellulose en glucose nécessite l'action synergique de trois catégories d'enzymes, les cellulases, classées en fonction de leur activité :
- les endoglucanases coupent la cellulose aléatoirement au niveau des zones amorphes de la cellulose,
- les exoglucanases (ou cellobiohydrolases quand elles produisent du cellobiose) agissent de façon progressive sur les extrémités libres des chaînes de cellulose, libérant du glucose ou du cellobiose,
- les β-glucosidases (ou cellobiases) hydrolysent les cellodextrines solubles et le cellobiose en glucose.

Un des principaux verrous limitant actuellement le développement industriel de ce procédé est le coût élevé associé à la production des cellulases. Afin d'améliorer l'activité des cocktails enzymatiques commerciaux, une supplémentation des cellulases avec des β-glucosidases est décrite dans la littérature, de façon à annihiler l'effet inhibiteur du cellobiose bien connu sur l'activité des endo- et exoglucanases.

Afin de réduire la quantité d'enzymes mises en oeuvre, différentes solutions ont été proposées pour pouvoir recycler ces enzymes. A la fin de l'hydrolyse enzymatique, les enzymes se retrouvent en partie sous forme libre dans l'hydrolysat et sous forme liée au résidu solide.

Dans le brevet FR-B-2 608 625 au nom de la Demanderesse, une méthode consistant à récupérer la majeure partie des enzymes est proposée : les enzymes libres sont récupérées par adsorption sur le substrat neuf à convertir, tandis que les enzymes liées sont réutilisées par recontactage du résidu solide avec le substrat neuf. Dans le cas où les cellulases sont supplémentées avec des β-glucosidases pour améliorer l'activité du cocktail enzymatique, la méthode proposée n'est toutefois pas satisfaisante : après recyclage, une perte d'activité importante est observée, associée à l'accumulation de cellobiose (Ramos et al, Applied Biochemistry and Biotechnology, 1994, 45 (6), 193-207). Cette méthode ne permet pas en effet de recycler efficacement les β-glucosidases libres qui n'ont que très peu d'affinité avec le substrat lignocellulosique.

La supplémentation des cellulases commerciales avec des β-glucosidases supportées est une méthode décrite par Woodward et al ("Use of immobilized beta-glucosidase in the hydrolysis of cellulose", 1993, ACS symposium series, 533, 240-250) qui permet de réutiliser plusieurs fois les β-glucosidases, sans perte d'activité ni diminution appréciable de la conversion de la cellulose en glucose. L'immobilisation des β-glucosidases permet d'améliorer notablement leur stabilité : Aguado et al. (Biotechnology and Applied Biochemistry, 1995, 17(1), 49-55) ont montré que l'immobilisation des β-glucosidases de *Penicillium funiculosum* sur de la poudre de nylon permettait d'obtenir une activité stable pendant 1500 min à 50°C alors que ces mêmes enzymes à l'état libre se désactivent à partir de 40°C. Les β-glucosidases *d'Aspergillus niger* sont quant à elles stables à l'état libre jusqu'à 45°C, tandis que sous la forme immobilisée sur Eupergit C, leur activité reste stable jusqu'à 65°C (Tu et al, 2006, Biotechnology Letters, 28 (3), 151-156). A ces températures, les cellulases sont en revanche peu stables, et se désactivent rapidement, ce qui impacte l'efficacité de l'hydrolyse.

Le recyclage des β-glucosidases supportées nécessitent de les extraire du mélange réactionnel contenant un résidu solide, ce qui ne peut pas être réalisé aisément lorsque les cellulases et les β-glucosidases sont utilisées dans le même réacteur.

D'autre part, l'adsorption non productive des β-glucosidases sur la lignine est une source de limitation connue de l'hydrolyse enzymatique. Une des solutions connues pour limiter cette adsorption consiste à ajouter des surfactants et/ou des protéines tels que l'albumine de sérum bovin (ASB) (Yang et al., 2006, Biotechnology and Bioengineering, 94 (4), 611-617).

Ces principales limitations peuvent être levées en réalisant la mise en oeuvre du procédé de jus sucré selon la présente invention.

Le document US5962289 décrit des protéines de fusion se liant à la cellulose. L'exemple 5 décrit la production de glucose à partir de cellobiose en utilisant des protéines de fusion de β-glucosidases immobilisées. Des endo- et exo-glucanases sont ajoutées au milieu comprenant le matériau cellulosique à dégrader. La totalité du mélange est ensuite envoyée vers une colonne Avicel, qui immobilise et concentre les endo- et les exo-glucanases. L'éluent contenant le cellobiose est envoyé vers une seconde colonne Avicel contenant des β-glucosidases immobilisés, le cellobiose étant ainsi converti en glucose.

Le document EP0062027 décrit un procédé de production de monosaccharides à partir d'une matière première cellulosique comprenant les étapes suivantes:
le matériau cellulosique est mélangé à une solution de cellulases avant d'être envoyé dans une série de premiers réacteurs d'hydrolyse enzymatique placés en parallèle. La totalité de l'hydrolysat obtenu, donc comprenant une phase liquide mélangée à des résidus solides, passe clans un agitateur et un moulin avant d'être envoyé dans une centrifugeuse permettant de séparer les résidus solides de la phase liquide. La phase liquide est alors envoyée dans un deuxième réacteur comprenant des β-glucosidases supportées. Le flux sortant de ce réacteur est ensuite recirculé en partie vers les premiers réacteurs d'hydrolyse ou vers l'agitateur, ou il est mélangé avec un microorganisme pour l'étape de fermentation des jus sucrés.

### Résumé de l'invention

Le procédé selon la présente invention consiste à produire un jus sucré à partir de substrat lignocellulosique par hydrolyse enzymatique comprenant une phase de conversion de la lignocellulose, une phase de production de jus sucré et une phase de vidange complète des réacteurs, dans lequel on réalise la supplémentation des cellulases avec des β-glucosidases supportées dans un réacteur (2) séparé du réacteur (1) d'hydrolyse enzymatique de la lignocellulose, ledit substrat, préalablement prétraité, étant mis en contact avec une solution de cellulases, l'hydrolysat H1 produit lors de la phase de conversion et obtenu en sortie du réacteur (1) étant envoyé dans un média filtrant (3) et dans un moyen de séparation de façon à ce que la fraction solide et les cellulases présentent dans le mélange réactionnel restent dans le réacteur (1), avant d'être envoyé dans le réacteur (2), la majorité du flux d'hydrolysat (H2) issu du réacteur (2) étant réinjectée vers le réacteur (1) pendant la phase de conversion.

La présente invention décrit également le dispositif dans lequel le procédé d'hydrolyse enzymatique est réalisé.

### Description des figures

La Figure 1 représente un schéma du procédé selon la présente invention correspondant à la phase de conversion de la lignocellulose.
La Figure 2 représente un schéma du procédé selon la présente invention correspondant à la phase de production continue de jus sucrés.
Les Figures 3 correspondent aux schémas du procédé de production de jus sucrés selon l'invention mis en oeuvre en mode discontinu, la Figure 3A correspondant à la phase de vidange des deux réacteurs et la Figure 3B à la phase de remplissage des réacteurs.

La Figure 4 représente un schéma du procédé selon la présente invention correspondant à la phase de vidange complète du réacteur d'hydrolyse enzymatique de lignocellulose.

### Description détaillée de l'invention

La présente invention concerne un procédé de production de jus sucré à partir de substrat lignocellulosique par hydrolyse enzymatique comprenant une phase de conversion de la lignocellulose, une phase de production de jus sucré et une phase de vidange complète des réacteurs, dans lequel on réalise la supplémentation des cellulases avec des β-glucosidases supportées dans un réacteur (2) séparé du réacteur (1) d'hydrolyse enzymatique de la lignocellulose, ledit substrat, préalablement prétraité, étant mis en contact avec une solution de cellulases, l'hydrolysat H1 produit lors de la phase de conversion et obtenu en sortie du réacteur (1) étant envoyé dans un média filtrant (3) et dans un moyen de séparation de façon à ce que la fraction solide et les cellulases présentent dans le mélange réactionnel restent dans le réacteur (1), avant d'être envoyé dans le réacteur (2), la majorité du flux d'hydrolysat (H2) issu du réacteur (2) étant réinjectée vers le réacteur (1) pendant la phase de conversion.

Le procédé selon l'invention permet notamment d'améliorer le recyclage des enzymes, en facilitant l'extraction des β-glucosidases du milieu réactionnel.

Un autre avantage du procédé selon la présente invention est de pouvoir dissocier les conditions de fonctionnement (température et éventuellement pH) des deux réacteurs et par conséquent de les optimiser dans le deuxième réacteur propre aux β-glucosidases. Les cellulases sont extraites en sortie de réacteur (1) et ne sont pas envoyés dans le deuxième réacteur.

De plus, dans le procédé de la présente invention, les β-glucosidases supportées ne sont pas en contact avec la lignine qui reste dans le réacteur dédié à l'hydrolyse enzymatique de la lignocellulose.

Le procédé selon la présente invention permet de produire un jus sucré, riche en glucose à partir de substrats lignocellulosiques.

Les substrats utilisés sont choisis parmi les pailles, le bois, les cultures forestières, les résidus de plantes alcooligènes, sucrières et céréalières, les résidus de l'industrie papetière et les produits de transformation des matériaux cellulosiques et lignocellulosiques.

Dans le procédé selon la présente invention, l'hydrolysat H1 obtenu en sortie de réacteur 1 est envoyé vers une membrane d'ultrafiltration (7) permettant d'extraire les cellulases.

Le procédé de production de jus sucré selon la présente invention comprend une phase de conversion de la lignocellulose, une phase de production proprement dite du jus sucré et une phase de vidange complète des réacteurs.

Pour mettre en oeuvre le procédé selon la présente invention, on met en contact dans le réacteur (1) un substrat lignocellulosique (S), préalablement prétraité, avec une solution de cellulases (E) dans des conditions de dilution, de température et de pH favorables à l'hydrolyse enzymatique de la lignocellulose. Le prétraitement réalisé selon les techniques connues par l'homme de l'art permet d'améliorer la susceptibilité du substrat pour l'hydrolyse enzymatique.

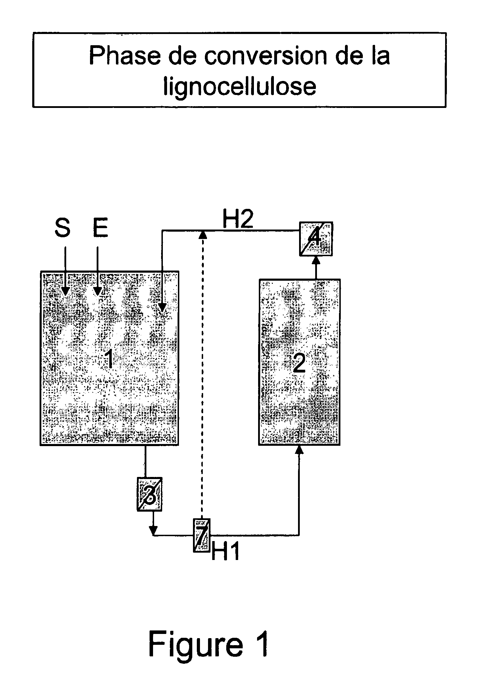

Lorsque le substrat commence à se liquéfier (Figure 1), la phase de conversion de la lignocellulose produit un hydrolysat extrait en continu du réacteur (1) dans un média filtrant (3) et dans un moyen de séparation (7) de façon à ce que la fraction solide (résidu solide de l'hydrolyse enzymatique) et les cellulases présentes dans le mélange réactionnel restent dans le réacteur (1). Après passage dans le média filtrant (3) et le moyen de séparation (7), l'hydrolysat (H1), contenant des oligomères solubles de glucose non fermentescibles, est injecté dans le réacteur (2) où il est mis en contact avec des β-glucosidases supportées, dans des conditions de température favorables à l'hydrolyse des oligomères solubles de glucose (cellobiose, cellotriose...) en glucose. Le temps de séjour du jus sucré (H1) dans le réacteur (2) est ajusté de façon à ce que la teneur en oligomères solubles résiduelles soit très faible (de préférence inférieure à 1 g/L) dans l'hydrolysat obtenu en sortie du réacteur (2).

La température dans le réacteur (1) est comprise préférentiellement entre 40 et 55°C et celle dans le réacteur (2) entre 40 et 70°C.

Les conditions opératoires de température et/ou de pH des réacteurs (1) et (2) peuvent être différentes ou identiques.

De façon très préférée, les conditions opératoires des réacteurs (1) et (2) sont différentes. Très préférentiellement, la température dans le réacteur (1) est comprise entre 45 et 55°C et la température dans le réacteur (2) est comprise entre 60 et 70°C.

Le moyen de séparation (7) est préférentiellement une membrane d'ultrafiltration. En sortie de cette membrane, le perméat débarrassé des cellulases est envoyé dans le réacteur (2), alors que le rétentat est mélangé au flux (H2) sortant du réacteur (2). Ce mode de réalisation permet notamment de faire fonctionner le réacteur (2) par exemple à des températures plus élevées que celles couramment utilisées pour les cellulases.

L'hydrolysat pauvre en oligomères solubles (H2) est extrait en continu du réacteur (2) dans un média filtrant (4), de façon à ce que les β-glucosidases supportées présentes dans le mélange réactionnel restent dans le réacteur (2) : le choix du média filtrant (4) se fait en fonction de la granulométrie du support choisi pour immobiliser les enzymes supportées.

Lors de la phase de conversion, la majorité du flux (H2), et préférentiellement la totalité du flux (H2) produit est réinjecté dans le réacteur (1).

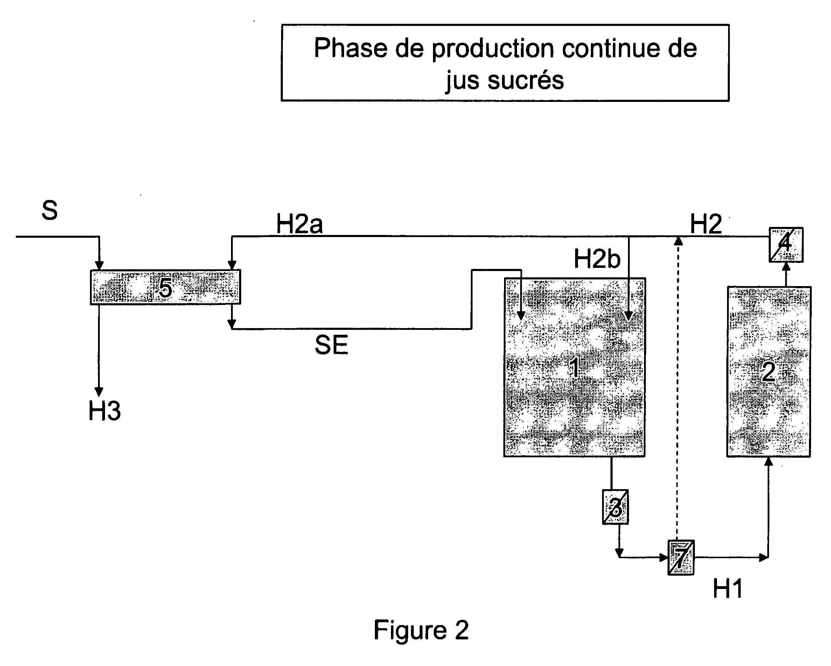

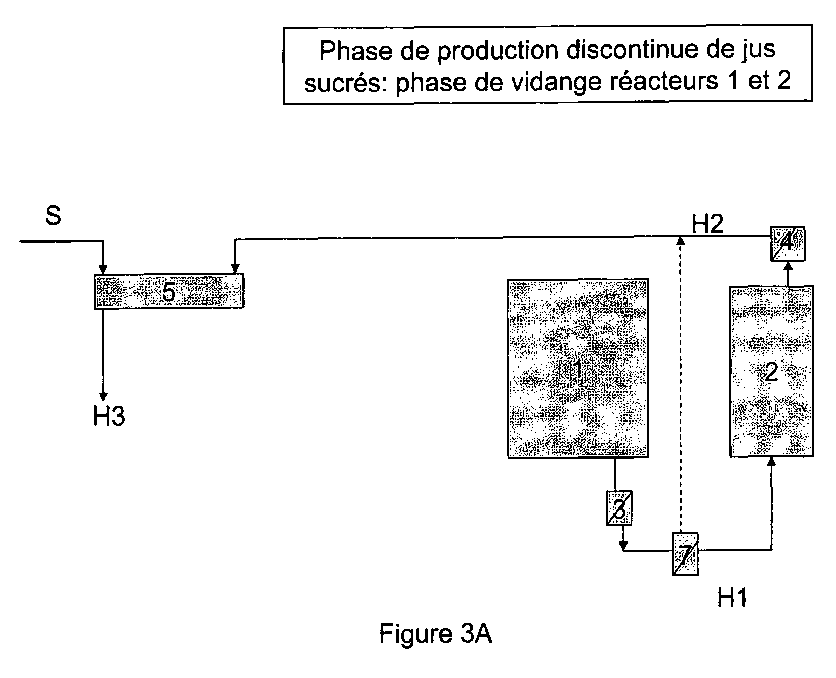

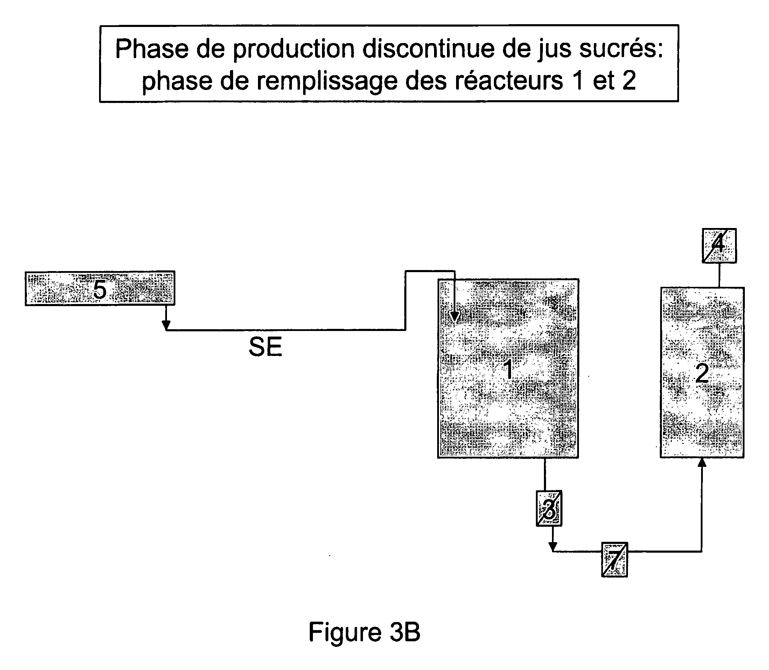

Lorsque la conversion est jugée suffisante, c'est-à-dire lorsque le dosage réalisé indique que l'on atteint la concentration souhaitée en sucres dans l'hydrolysat (H2), on démarre la phase de production proprement dite de jus sucrés, illustrée par les Figures 2 et 3.

La phase de production du jus sucré peut se faire selon deux modes de réalisation : en mode continu ou en mode discontinu.

Selon le premier mode de réalisation, la phase de production est réalisée en mode continu. Le flux d'hydrolysat (H2) issu du réacteur (2) est séparé en au moins deux flux (H2a) et (H2b).

Le flux (H2a) est introduit en continu dans un contacteur (5). Cet hydrolysat (H2a) est mis en contact dans l'équipement (5) avec du substrat lignocellulosique neuf (S) de façon à ce que les cellulases présentes dans l'hydrolysat (H2a) s'adsorbent sur le substrat neuf.

Le flux (H2b), pauvre en oligomères solubles, est réinjecté dans le réacteur (1) permettant ainsi d'améliorer les conditions de mélange dans le réacteur et de favoriser l'activité des cellulases par l'effet diluant induit, les oligomères solubles, étant en effet connus comme produits inhibiteurs de l'activité des cellulases.

Le substrat neuf imprégné de cellulases (SE), issu du contacteur (5), est séparé de l'hydrolysat (H3) avant d'être envoyé dans le réacteur (1).

L'hydrolysat (H3) récupéré à la sortie du contacteur (5) correspond au flux de jus sucrés produit par le procédé.

Selon le deuxième mode de réalisation du procédé selon l'invention, la phase de production est réalisée en mode discontinu et se fait en deux étapes (Figures 3A et 3B), la première étape consistant en la vidange des réacteurs, et la deuxième au redémarrage.

On procède dans un premier temps à la vidange des réacteurs : le flux (H2) issu du réacteur (2) est envoyé dans le contacteur (5), où il est mis en contact avec du substrat lignocellulosique neuf (S) de façon à ce que les cellulases introduites dans l'hydrolysat (H2) en sortie du moyen de séparation (7) s'adsorbent sur le substrat neuf. L'hydrolysat (H3) obtenu en sortie du contacteur correspond au flux de jus sucré produit par le procédé.

À la fin de la phase de vidange des réacteurs, on procède au redémarrage de la phase de production en mode discontinu (Figure 3B), en introduisant le substrat neuf imprégné de cellulases (SE) dans le réacteur (1).

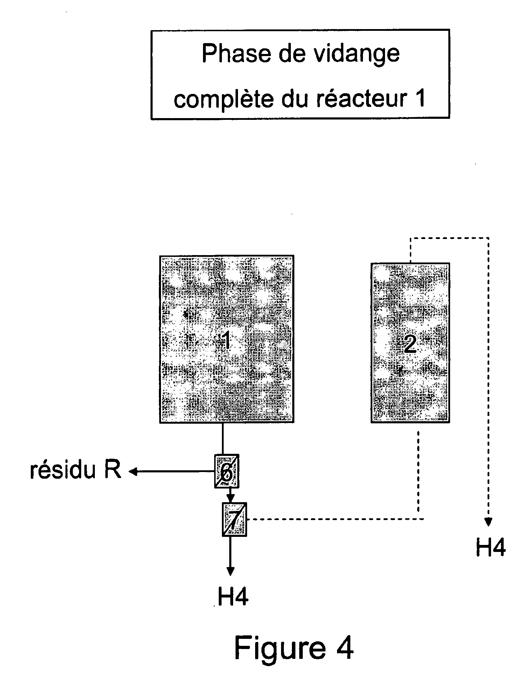

Lorsque la quantité de résidu solide accumulée dans le réacteur (1) devient trop importante, on procède à la vidange complète du réacteur (1) (Figure 4). Le mélange réactionnel récupéré est envoyé sur un média filtrant (6), de façon à séparer la fraction solide (résidu R), correspondant au résidu de l'hydrolyse enzymatique, puis vers le moyen de séparation (7) en sortie duquel on obtient une fraction liquide (H4).

Cette fraction liquide (H4) est un jus sucré. En vue d'optimiser la production en glucose, si cette fraction (H4) contient des oligomères solubles résiduels, il est possible de l'envoyer dans le réacteur (2) avant de l'extraire.

La fraction (H4), correspondant au flux produit par le procédé pendant la phase de vidange, peut éventuellement être en partie ou totalement réintroduite dans le réacteur (1) lors de la phase de démarrage de l'hydrolyse enzymatique en réintroduisant des cellulases fraîches et du substrat neuf ou être utilisée pour imprégner du substrat neuf dans le contacteur (5).

La présente invention décrit également le dispositif dans lequel le procédé de production de jus sucré décrit précédemment est mis en oeuvre.

Ledit dispositif pour la production de jus sucré par hydrolyse enzymatique à partir de substrat lignocellulosique comporte :
- au moins un premier réacteur (1) d'hydrolyse enzymatique de la lignocellulose, ledit réacteur (1) étant muni d'au moins une conduite pour l'introduction de substrat et de la solution de cellulases, d'au moins une conduite pour soutirer le jus converti (H1) et l'envoyer vers au moins un média filtrant (3) placé en sortie du réacteur (1) et d'au moins un moyen de séparation (7), qui est préférentiellement une membrane d'ultrafiltration, pour extraire les cellulases de l'hydrolysat,
- éventuellement un échangeur thermique en sortie du premier réacteur (non représenté sur les figures),
- au moins un deuxième réacteur (2) comprenant les β-glucosidases supportées, relié audit moyen de séparation (7) par au moins une conduite, ledit réacteur (2) étant équipé en sortie d'au moins un média filtrant (4) et d'au moins une conduite pour l'extraction du flux (H2) constitué de l'hydrolysat pauvre en oligomères solubles,
- au moins une conduite pour introduire au moins une partie du flux (H2) dans un contacteur (5) et au moins une conduite pour renvoyer au moins une partie du flux (H2) dans le réacteur (1),
- au moins un contacteur (5), muni d'au moins une conduite pour l'introduction du substrat neuf, d'au moins une conduite pour l'extraction du jus sucré produit (H3) et d'au moins une conduite pour la sortie du substrat neuf imprégné de cellulases et son introduction dans le réacteur (1).

Ledit dispositif comporte au moins un média filtrant (6) utilisé lors de la phase de vidange complète du réacteur (1), au moins une conduite pour l'extraction du résidu solide R, au moins une conduite pour l'extraction du flux (H4) de jus sucré produit et éventuellement au moins une conduite pour l'introduction du flux (H4) dans le réacteur (2).

Le réacteur (1) peut être une cuve mécaniquement agitée, ou non. L'agitation peut être également entièrement ou en partie assurée par la boucle de circulation du jus sucré autour du réacteur (1) (flux H2b ou flux H2), en utilisant des fortes vitesses d'injection.

Les médias filtrants (3) et (6) permettant d'extraire la fraction liquide du mélange réactionnel sont adaptés à la granulométrie du substrat lignocellulosique solide. Tout équipement connu de l'homme de l'art peut être utilisé pour réaliser ce fractionnement : système de filtration installé à l'intérieur ou à l'extérieur du réacteur incluant le système de rétro-lavage ou nettoyage associé (bougies filtrantes, grille mécaniquement raclée...), module de séparation externe avec réinjection de la fraction solide dans le réacteur (filtre-presse, centrifugeuse...). Le dimensionnement de ces équipements doit être adapté au volume à traiter.

Ces médias filtrants (3) et (6) peuvent éventuellement être confondus avec le moyen de séparation (7).

La mise en oeuvre des enzymes immobilisées dans le réacteur (2) peut être réalisée en lit fixe ou en lit mobile ou dans un réacteur de type slurry agité. Le média filtrant (4) a pour objet de maintenir les enzymes supportées dans le réacteur (2).

Dans le cas d'un lit fixe, qui est la mise en oeuvre préférée, ce média (4) peut être une grille ou un lit de garde installé dans le réacteur.

Pour les autres modes de réalisation (lit mobile ou réacteur de type slurry agité), les équipements décrits précédemment pour le média filtrant (3) peuvent convenir également au média filtrant (4).

Pour pallier la désactivation des β-glucosidases supportées, un appoint continu d'enzymes fraîches couplé à un soutirage d'enzymes désactivées peut être réalisé dans le cas des mises en oeuvre en lit mobile ou slurry. Dans le cas d'une mise en oeuvre en lit fixe, le remplacement des enzymes désactivées par des enzymes neuves se fait en mode discontinu lors de la phase de vidange des réacteurs (1) et (2) par exemple.

Le recyclage des cellulases effectué dans l'équipement (5) peut être réalisé par mise en contact de l'hydrolysat (H2a) ou (H4) avec du substrat lignocellulosique frais prétraité, ledit contact se faisant par exemple par percolation ou par remise en suspension et séparation, de manière à récupérer les cellulases par adsorption, selon toute méthode connue par l'homme de l'art. L'équipement (5) peut donc être un équipement similaire à celui décrit pour l'ensemble réacteur (1) et média filtrant (3), dans lesquels on peut effectuer la remise en suspension et filtration du substrat.

L'équipement (5) peut également être tout type de technologie permettant de réaliser un contactage liquide-solide par percolation du liquide sur le solide en mode discontinu ou préférentiellement en mode continu. Dans le cas d'une mise en contact par percolation, l'équipement (5) pourra intégrer en outre un système de filtre presse permettant de réduire la quantité de jus sucré qui sera réintroduite dans le réacteur (1).

La préparation des β-glucosidases supportées ou immobilisées est réalisée à partir de β-glucosidases produites par des souches de champignons appartenant aux genres *Trichoderma, Aspergillus, Penicillium* ou *Schizophyllum,* par exemple par *Aspergillus niger, Penicillium funiculosum,* ou par *Trichoderma reesei.* Ces souches peuvent avoir été génétiquement modifiées. Leur immobilisation est réalisée en utilisant tout type de support (solide ou gel) connu de l'homme de l'art (résines, Eupergit C, nanotubes de carbone activé, alumine recouverte d'une matrice polymère, poudre de nylon activée, alginate de calcium, chitosan...).

Les exemples suivants illustrent la présente invention sans en limiter la portée.

### Exemple 1 : Hydrolyse enzymatique de paille de blé avec recyclage d'enzymes, selon l'enseignement du brevet FR-B-2 608 625 (β-glucosidases non supportées)

Un échantillon de paille de blé a été prétraité par explosion à la vapeur d'eau en présence d'acide sulfurique de façon à améliorer sa digestibilité, c'est-à-dire sa réactivité vis-à-vis de l'hydrolyse enzymatique. La composition de cet échantillon prétraité et lavé est la suivante : 56,5% de cellulose, 14,6% d'hemicellulose, 25,6% de lignine et composés extractibles (en % matière sèche). L'hydrolyse enzymatique de cet échantillon a été réalisée en présence de cellulases commerciales de SAF-ISIS (référence XL-E508) complémentées avec des β-glucosidases commercialisées par Novozyme (référence SP-188). La quantité de cellulases utilisées dans les essais est exprimée en unités internationales "papier filtre" (uPF), celles des β-glucosidases en unités internationales "Ul" (1UI= 1 µmol de glucose produite par min à partir de cellobiose).

Une première série d'essais (A1, B1, C1 correspondant respectivement aux essais A, B et C réalisés dans les conditions 1) a été réalisée dans 3 réacteurs agités contenant chacun 100 g de substrat (exprimé en matière sèche), 480 uFP (cellulases), 1000 ul (β-glucosidases) dans un volume final de 1 litre. Le pH a été ajusté à 4,8 et la température à 50°C. Après 72 heures de temps d'hydrolyse, les résidus solides ont été séparés par centrifugation et les sucres formés dans l'hydrolysat ont été dosés par chromatographie liquide à haute performance (HPLC). Une deuxième série d'essais (A2, B2, C2) a été réalisée dans des conditions identiques à celles de la première série d'essais, sauf en ce qui concerne les points suivants :
- l'essai B2 a été conduit sans ajout d'enzymes fraîches mais les enzymes solubles ont été recyclées par recontactage de l'hydrolysat obtenu à la fin de l'essai B1 avec le substrat neuf et 100% du résidu solide obtenu à la fin de l'essais B1 a été recyclé,
- l'essai C2 a été conduit à l'identique de l'essai B2, en ajoutant 1000 ul de β-glucosidases fraîches.

Une troisième et dernière série d'essais (A3, B3, C3) a été réalisée dans des conditions rigoureusement identiques à celles de la deuxième série d'essais.

Les résultats obtenus sont présentés dans le tableau 1. Les sucres produits après chaque série d'essais sont mesurés à partir du dosage des sucres présents dans l'hydrolysat final. Pour les séries B et C, la quantification des sucres produits au cours des 2^{ème} et 3^{ème} séries d'essais est réalisée en décomptant les sucres présents au démarrage de l'hydrolyse enzymatique (sucres contenus dans le résidu solide).

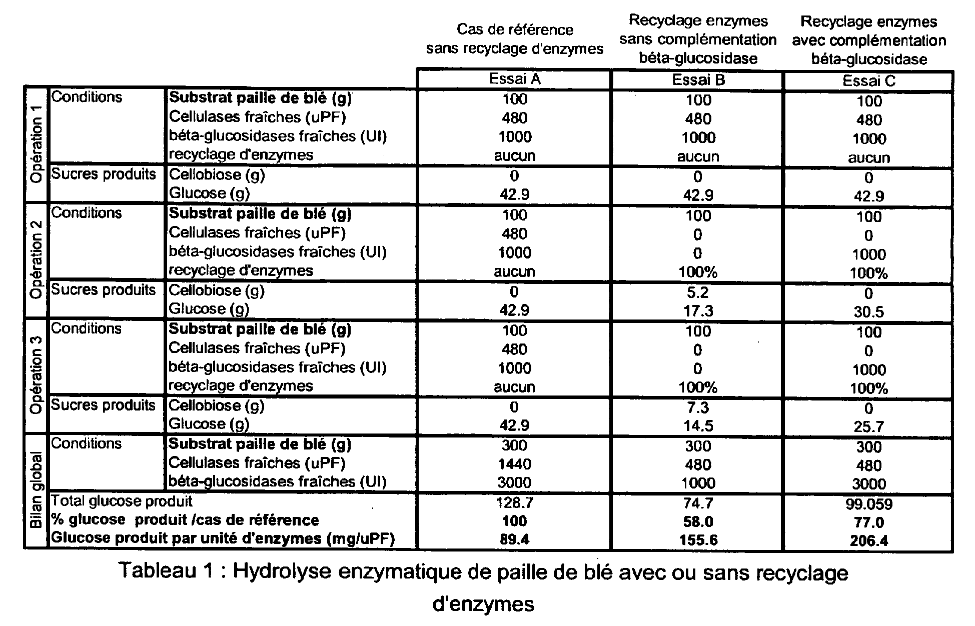

Les résultats de la série d'essais B, correspondant à la méthode décrite dans le brevet FR-B-2 608 625; montrent que les performances obtenues au cours des opérations 2 et 3 sont nettement dégradées : la production de glucose à l'issu des 3 opérations d'hydrolyse ne représente que 58% du cas de référence. Un ajout de β-glucosidases neuves au cours des opérations 2 et 3, réalisé pour la série d'essais C permet d'améliorer les performances : la production de glucose à l'issu des 3 opérations d'hydrolyse représente 77% du cas de référence (essai A).

### Exemple 2: Hydrolyse enzymatique de paille de blé avec recyclage d'enzymes et utilisation de β-glucosidases supportées dans un réacteur séparé selon le procédé décrit dans la présente invention

Une série d'essais d'hydrolyse a été réalisée avec la même paille de blé que dans l'Exemple 1.

Une première opération d'hydrolyse D1 a été réalisée dans un réacteur agité contenant 100g de substrat (exprimé en matière sèche) et 480 uFP (cellulases) dans un volume final de 1 litre. Le pH a été ajusté à 4,8 et la température à 50°C. Après 6 heures de temps d'hydrolyse, la fraction liquide du mélange réactionnel est extraite en continu à travers un média filtrant, type membrane d'ultrafiltration à un débit de 10 ml/min pour introduire en continu l'hydrolysat sans cellulases à l'aide d'une pompe dans un deuxième réacteur rempli d'un lit fixe de β-glucosidases supportées sur Eupergit C, préparé selon le protocole décrit par Tu et al contenant 1000 ul (activité β-glucosidases). La température du deuxième réacteur est maintenue à 60°C. L'intégralité du flux sortant de ce deuxième réacteur est réinjectée dans le premier réacteur d'hydrolyse enzymatique. Après 66 heures d'opération, on vidange les deux réacteurs. Le résidu solide est séparé par centrifugation et les sucres formés dans l'hydrolysat ont été dosés par HPLC.

Une deuxième opération D2 a été réalisée dans des conditions identiques à celles de D1, sauf en ce qui concerne les points suivants :
- l'essai D2 a été conduit sans ajout d'enzymes fraîches mais les enzymes solubles ont été recyclées par recontactage de l'hydrolysat obtenu à la fin de l'essai D1 avec le substrat neuf et 100% du résidu solide obtenu à la fin de l'essai D1 a été recyclé. Une troisième opération D3 a été réalisée dans des conditions rigoureusement identiques à celle de l'essai D2.

Les résultats obtenus sont présentés dans le tableau 2.

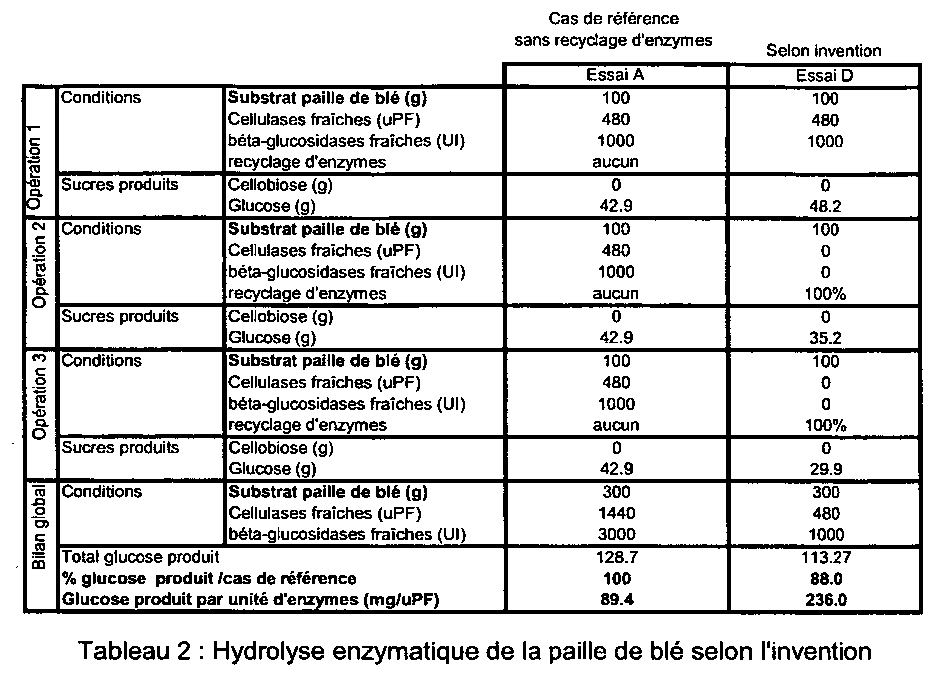

L'utilisation de β-glucosidases supportées mise en oeuvre dans un réacteur séparé permet d'améliorer nettement les performances : la production de glucose à l'issu des 3 opérations d'hydrolyse représente 88% du cas de référence (essai A).

### Exemple 3 : Hydrolyse enzymatique de pâte à papier avec recyclage d'enzymes, selon l'enseignement du brevet FR-B-2 608 625 (β-glucosidases non supportées)

Les séries d'essais décrites dans l'Exemple 1 ont été reconduites avec un échantillon de pâte à papier non blanchi. La composition de cet échantillon est la suivante : 70,3% de cellulose, 15,9% d'hemicellulose, 7,6% de lignine et composés extractibles (en % matière sèche). Les résultats obtenus, après 48 heures de temps d'hydrolyse, sont présentés dans le tableau 3.

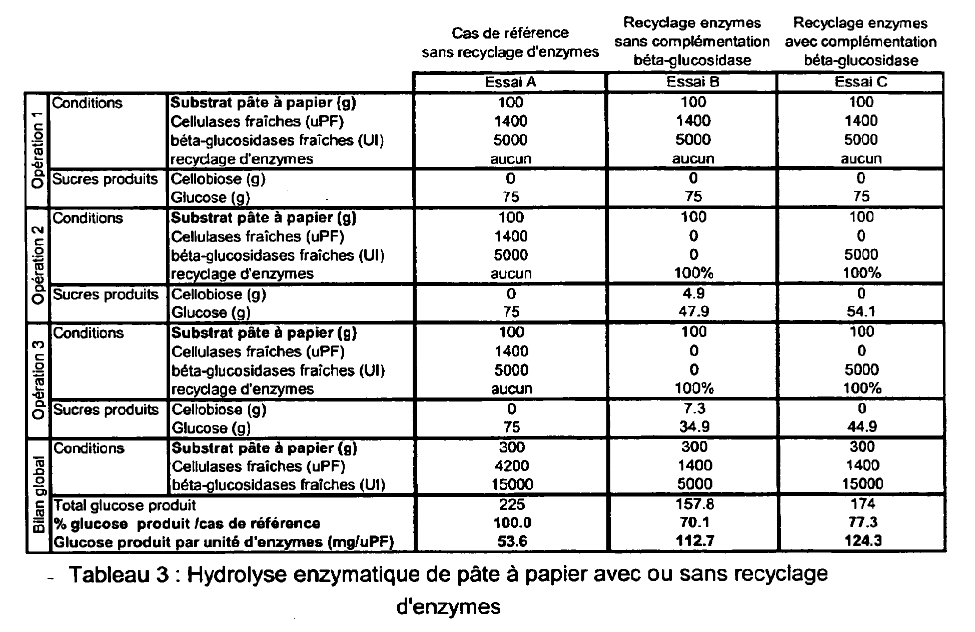

Les résultats de la série d'essais B, correspondant à la méthode décrite dans le brevet FR-B-2,608,625, montrent que les performances obtenues au cours des opérations 2 et 3 sont nettement dégradées : la production de glucose à l'issu des 3 opérations d'hydrolyse ne représente que 70,1 % du cas de référence. Un ajout de β-glucosidases neuves au cours des opérations 2 et 3, réalisé pour la série d'essais C permet d'améliorer les performances : la production de glucose à l'issu des 3 opérations d'hydrolyse représente 77,3% du cas de référence (essai A).

### Exemple 4: Hydrolyse enzymatique de pâte à papier avec recyclage d'enzymes et utilisation de β-glucosidases supportées dans un réacteur séparé selon le procédé décrit dans la présente invention

Une série d'essais d'hydrolyse enzymatique a été réalisée avec la même pâte à papier que dans l'Exemple 3 et dans des conditions de mises en oeuvre strictement identiques à celles décrites dans l'Exemple 2, avec un temps d'hydrolyse limité à 48 heures et des quantités d'enzymes ajustées pour le nouveau substrat choisi (1400 uPF de cellulases et 5000 Ul de β-glucosidases immobilisées).

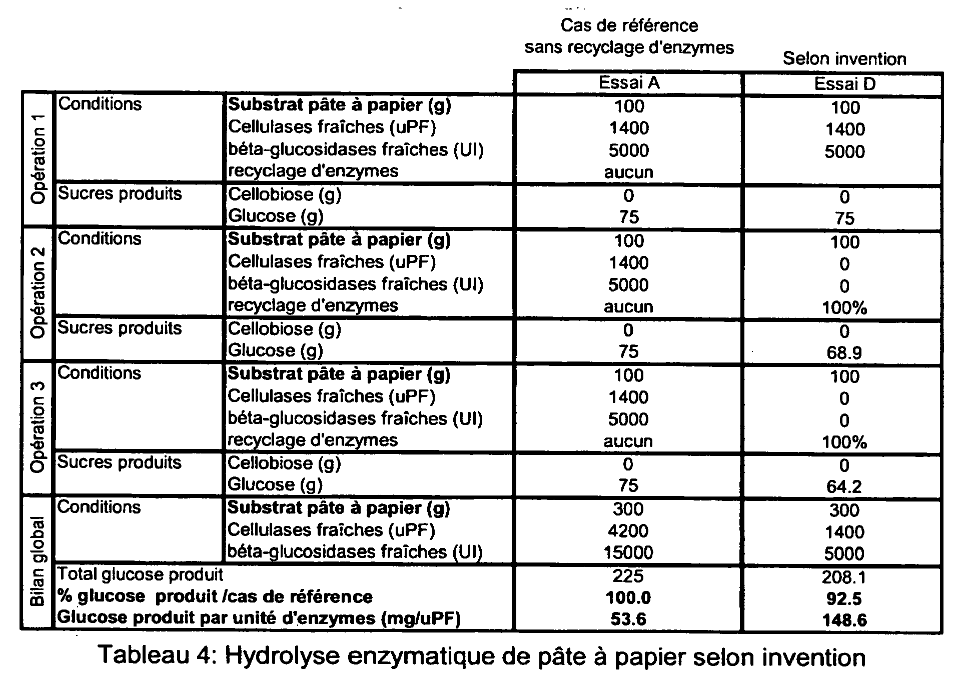

L'utilisation de β-glucosidases supportées mise en oeuvre dans un réacteur séparé permet là encore d'améliorer nettement les performances : la production de glucose à l'issu des 3 opérations d'hydrolyse représente 92,5% du cas de référence (essai A).

## Revendications

1. Procédé de production de jus sucré à partir de substrat lignocellulosique par hydrolyse enzymatique comprenant une phase de conversion de la lignocellulose, une phase de production de jus sucré et une phase de vidange complète des réacteurs, dans lequel on réalise la supplémentation des cellulases avec des β-glucosidases supportées dans un réacteur (2) séparé du réacteur (1) d'hydrolyse enzymatique de la lignocellulose, ledit substrat, préalablement prétraité, étant mis en contact avec une solution de cellulases, l'hydrolysat H1 produit lors de la phase de conversion et obtenu en sortie du réacteur (1) étant envoyé dans un média filtrant (3) et dans un moyen de séparation de façon à ce que la fraction solide et les cellulases présentent dans le mélange réactionnel restent dans le réacteur (1), avant d'être envoyé dans le réacteur (2), la majorité du flux d'hydrolysat (H2) issu du réacteur (2) étant réinjectée vers le réacteur (1) pendant la phase de conversion.

2. Procédé selon la revendication 1 dans lequel le moyen de séparation (7) est une membrane d'ultrafiltration.

3. Procédé selon l'une des revendications 1 ou 2 dans lequel la totalité du flux d'hydrolysat (H2) issu du réacteur (2) est réinjectée vers le réacteur (1) pendant la phase de conversion.

4. Procédé selon l'une des revendications 1 à 3 dans lequel la phase de production de jus sucrés est réalisée selon un mode continu ou discontinu.

5. Procédé selon l'une des revendications 1 à 4 dans lequel la phase de production de jus sucrés est réalisée selon un mode continu, l'hydrolysat (H2), issu du réacteur (2) et comprenant le rétentat issu du moyen de séparation (7), étant séparé en au moins deux flux (H2a) et (H2b), le flux (H2a) étant introduit en continu dans un contacteur (5) et le flux (H2b) étant réinjecté dans le réacteur (1).

6. Procédé selon la revendication 5 dans lequel le flux (H2a), est mis en contact avec du substrat lignocellulosique neuf dans l'équipement (5).

7. Procédé selon la revendication 6 dans lequel le substrat neuf imprégné de cellulases (SE) issu du contacteur (5) est séparé de l'hydrolysat (H3) avant d'être envoyé dans le réacteur (1).

8. Procédé selon l'une des revendication 1 à 4 dans lequel la phase de production de jus sucrés est réalisée selon un mode discontinu, une première étape consistant en la vidange des réacteurs au cours de laquelle le flux d'hydrolysat (H2) issu du réacteur (2) est envoyé dans le contacteur (5) où il est mis en contact avec du substrat lignocellulosique neuf et une deuxième étape consistant au redémarrage par introduction du substrat neuf imprégné de cellulases (SE) dans le réacteur (1).

9. Procédé selon l'une des revendications 1 à 8 dans lequel, pendant la phase de vidange complète du réacteur (1), le mélange réactionnel contenu dans le réacteur (1) est envoyé vers un média filtrant (6) d'où on sépare le résidu solide (R) , puis vers le moyen de séparation (7) en sortie duquel on obtient une fraction liquide (H4) constituée d'un jus sucré, éventuellement envoyée vers le réacteur (2)

10. Procédé selon la revendication 9 dans lequel la fraction liquide (H4) est réintroduite totalement ou en partie dans le réacteur (1) lors de la phase de démarrage de l'hydrolyse enzymatique en réintroduisant des cellulases fraîches et du substrat neuf.

11. Procédé selon la revendication 9 dans lequel la fraction liquide (H4) est envoyée vers le contacteur 5 pour imprégner du substrat neuf.

12. Procédé selon l'une des revendications 1 à 11 dans lequel les conditions opératoires de température et/ou de pH des réacteurs (1) et (2) sont différentes .

13. Dispositif pour la production de jus sucré par hydrolyse enzymatique à partir de substrat lignocellulosique comportant :
- au moins un premier réacteur (1) d'hydrolyse enzymatique de la lignocellulose, ledit réacteur (1) étant muni d'au moins une conduite pour l'introduction de substrat et de la solution de cellulases, d'au moins une conduite pour soutirer le jus converti H1 et l'envoyer vers au moins un média filtrant (3) placé en sortie du réacteur (1), au moins un moyen de séparation (7) pour extraire les cellulases de l'hydrolysat,
- éventuellement un échangeur thermique en sortie du premier réacteur,
- au moins un deuxième réacteur (2) comprenant les β-glucosidases supportées, relié à au moins un moyen de séparation (7), par au moins une conduite, ledit réacteur (2) étant équipé en sortie d'au moins un média filtrant (4) et d'au moins une conduite pour l'extraction du flux H2 constitué de l'hydrolysat pauvre en oligomères solubles.
- au moins une conduite pour introduire au moins une partie du flux H2 dans un contacteur (5) et au moins une conduite pour renvoyer au moins une partie du flux H2 dans le réacteur (1),
- au moins un contacteur (5), muni d'au moins une conduite pour l'introduction du substrat neuf, d'au moins une conduite pour l'extraction du jus sucré produit (H3) et d'au moins une conduite pour la sortie du substrat neuf imprégné de cellulases et son introduction dans le réacteur (1).

14. Dispositif selon la revendication 13 **caractérisé en ce qu'**il comporte au moins un média filtrant (6) utilisé lors de la phase de vidange complète du réacteur (1), au moins une conduite pour l'extraction du résidu solide R, au moins une conduite pour l'extraction du flux H4 de jus sucré produit et éventuellement au moins une conduite pour l'introduction du flux H4 dans le réacteur (2).

15. Dispositif selon l'une des revendications 13 ou 14 dans lequel le réacteur (2) comprenant les β-glucosidases est un réacteur en lit mobile ou de type slurry agité, ou un réacteur en lit fixe.

16. Dispositif selon la revendication 15 dans lequel le média filtrant (4) est une grille ou un lit de garde installé dans le réacteur (2).

## Claims

1. A method of producing sweet juice from lignocellulosic substrate by enzymatic hydrolysis comprising a lignocellulose conversion stage, a sweet juice production stage and a stage of complete emptying of the reactors, wherein cellulase supplementation is carried out with supported β-glucosidases, in a reactor (2) separate from lignocellulose enzymatic hydrolysis reactor (1), wherein said substrate, which has been pretrcated beforehand, is contacted with a cellulase solution, hydrolysate H1 obtained at the outlet of reactor (1) being sent to a filter medium (3) and to a separation means so that the solid fraction and the cellulases present in the reaction mixture remain in reactor (1) prior to being sent to reactor (2), the major part of hydrolysate stream (H2) from reactor (2) being reinjected into reactor (1) during conversion stage.

2. A method as claimed in claim 1, wherein the separation means (7) is an ultrafiltration membrane.

3. A method as claimed in any one of claims 1 or 2, wherein all of hydrolysate stream (II2) from reactor (2) is reinjected into reactor (1) during conversion stage.

4. A method as claimed in any one of claims 1 to 3, wherein the sweet juice production stage is carried out in continuous or batch mode.

5. A method as claimed in any one of claims 1 to 4, wherein the sweet juice production stage is carried out in continuous mode, hydrolysate (H2) from the reactor (2) and comprising the retentate from separation means (7) being separated into at least two streams (H2a) and (H2b), stream (H2a) being continuously fed into contactor (5) and stream (H2b) being reinjected into reactor (1).

6. A method as claimed in claim 5, wherein stream (H2a) is contacted with new lignocellulosic substrate in equipment (5).

7. A method as claimed in claim 6, wherein the new substrate impregnated with cellulases (SE) from contactor (5) is separated from hydrolysate (H3) prior to being sent to reactor (1).

8. A method as claimed in any one of claims 1 to 4, wherein the sweet juice production stage is carried out in batch mode, a first stage consisting in emptying the reactors, during which hydrolysate stream (H2) from reactor (2) is sent to contactor (5) where it is contacted with new lignocellulosic substrate, and a second stage consisting in starting up again by feeding new substrate impregnated with cellulases (SE) into reactor(1).

9. A method as claimed in any one of claims 1 to 8 wherein, during the stage of complete emptying of reactor (1), the reaction mixture contained in reactor (1) is sent to a filter medium (6) wherein solid residue (R) is separated, then to separation means (7) at the outlet of which a liquid fraction (H4) consisting of a sweet juice is obtained and possibly sent to reactor (2).

10. A method as claimed in claim 9, wherein liquid fraction (H4) is totally or partly reintroduced into reactor (1) during the enzymatic hydrolysis starting phase by reintroducing fresh cellulases and new substrate.

11. A method as claimed in claim 9, wherein liquid fraction (H4) is sent to contactor (5) to impregnate new substrate.

12. A method as claimed in any one of claims 1 to 11, wherein the temperature and/or pH operating conditions of reactors (1) and (2) are different.

13. A device intended for sweet juice production by enzymatic hydrolysis from lignocellulosic substrate comprising:
- at least a first lignocellulose enzymatic hydrolysis reactor (1), said reactor (1) being provided with at least one line for introducing the substrate and the cellulase solution, at least one line for withdrawing the converted juice (H1) and sending it to at least one filter medium (3) arranged at the outlet of reactor (1), and at least one separation means (7), that is preferably an ultrafiltration membrane for extracting the cellulases from the hydrolysate,
- possibly a heat exchanger at the outlet of the first reactor,
- at least a second reactor (2) comprising the supported β-glucosidascs, connected to at least one separation means (7) by at least one line, said reactor (2) being equipped at the outlet thereof with at least one filter medium (4) and at least one line for extracting stream (H2) consisting of the hydrolysate poor in soluble oligomers,
- at least one line for feeding at least part of stream (H2) into a contactor (5) and at least one line for sending back at least part of stream (H2) to reactor (1),
- at least one contactor (5) provided with at least one line for introducing the new substrate, at least one line for extracting the sweet juice produced (H3) and at least one line for discharging the cellulase-impregnated new substrate and feeding it into reactor (1).

14. A device as claimed in claim 13, **characterized in that** it comprises at least one filter medium (6) used during the stage of complete emptying of reactor (1), at least one line for extracting solid residue (R), at least one line for extracting the sweet juice stream (H4) produced and possibly at least one line for feeding stream (H4) into reactor (2).

15. A device as claimed in any one of claims 13 or 14, wherein reactor (2) comprising the β-glucosidases is a moving bed or stirred slurry type reactor, or a fixed bed reactor.

16. A device as claimed in claim 15, wherein filter medium (4) is a grate or a guard bed arranged in reactor (2).

## Patentansprüche

1. Verfahren zur Herstellung von zuckerhaltigem Saft aus einem Lignocellulose-Substrat durch enzymatische Hydrolyse, umfassend eine Phase der Umwandlung der Lignocellulose, eine Phase der Herstellung von zuckerhaltigem Saft und eine Phase der vollständigen Leerung der Reaktoren, wobei die Cellulasen in einem Reaktor (2), der von dem Reaktor (1) zur enzymatischen Hydrolyse der Lignocellulose getrennt ist, mit geträgerten β-Glucosidasen ergänzt werden, wobei das Substrat, welches im Vorfeld eine Vorbehandlung erfahren hat, mit einer Lösung von Cellulasen in Kontakt gebracht wird, wobei das Hydrolysat H1, welches in der Umwandlungsphase erzeugt wird und an der Austrittsöffnung des Reaktors (1) anfällt, einem Filtermedium (3) und einem Trennmittel zugeführt wird, sodass die feststoffliche Fraktion und die Cellulasen, welche in der Reaktionsmischung vorliegen, im Reaktor (1) verbleiben, bevor es in dem Reaktor (2) zugeführt wird, wobei der überwiegende Teil des Hydrolysatstoffstroms (H2), der aus dem Reaktor (2) stammt, während der Umwandlungsphase in den Reaktor (1) zurückgeführt wird.

2. Verfahren nach Anspruch 1, wobei es sich bei dem Trennmittel (7) um eine Ultrafiltrationsmembran handelt.

3. Verfahren nach einem der Ansprüche 1 oder 2, wobei die der gesamte Hydrolysatstoffstrom (H2), der aus dem Reaktor (2) stammt, während der Umwandlungsphase in den Reaktor (1) zurückgeführt wird.

4. Verfahren nach einem der Ansprüche 1 bis 3, wobei die Phase der Herstellung süßer Säfte auf kontinuierlich oder chargenweise durchgeführt wird.

5. Verfahren nach einem der Ansprüche 1 bis 4, wobei die Phase der Herstellung zuckerhaltiger Säfte kontinuierlich durchgeführt wird, wobei das Hydrolysat (H2), welches aus dem Reaktor (2) stammt und das Retentat umfasst, das aus dem Trennmittel (7) stammt, in mindestens zwei Stoffströme (H2a) und (H2b) aufgetrennt wird, wobei der Stoffstrom (H2a) kontinuierlich in eine Kontaktvorrichtung (5) eingeleitet wird und der Stoffstrom (H2b) in den Reaktor (1) zurückgeführt wird.

6. Verfahren nach Anspruch 5, wobei der Stoffstrom (H2a) in dem Gerät (5) mit neuem Lignocellulose-Substrat in Kontakt gebracht wird.

7. Verfahren nach Anspruch 6, wobei das neue, mit Cellulasen durchtränkte Substrat (SE), welches aus der Kontaktvorrichtung (5) vom Hydrolysat (H3) getrennt wird, bevor es dem Reaktor (1) zugeführt wird.

8. Verfahren nach einem der Ansprüche 1 bis 4, wobei die Phase der Herstellung zuckerhaltiger Säfte chargenweise durchgeführt wird, wobei ein erster Schritt darin besteht, die Reaktoren zu leeren, wobei der Hydrolysatstoffstrom (H2), welcher aus dem Reaktor (2) stammt, währenddessen der Kontaktvorrichtung (5) zugeführt wird, wo er mit neuem Lignocellulose-Substrat in Kontakt gebracht wird, und ein zweiter Schritt darin besteht, durch Einführen des neuen, mit Cellulasen durchtränkten Substrats (SE) in den Reaktor (1) einen Neubeginn zu erzielen.

9. Verfahren nach einem der Ansprüche 1 bis 8, wobei die Reaktionsmischung, welche im Reaktor (1) enthalten ist, während der Phase des vollständigen Leerens des Reaktors (1) einem Filtermedium (6) zugeführt wird, wo der feststoffliche Rückstand (R) abtrennt wird, und dann dem Trennmittel (7), an dessen Austrittsöffnung eine flüssige Fraktion (H4) erhalten wird, die aus süßem Saft besteht und möglicherweise dem Reaktor (2) zugeführt wird.

10. Verfahren nach Anspruch 9, wobei, in der Phase des Neubeginns der enzymatischen Hydrolyse durch erneute Einführung von frischen Cellulasen und neuem Substrat, die flüssige Fraktion (H4) vollständig oder teilweise in den Reaktor (1) zurückgeführt wird.

11. Verfahren nach Anspruch 9, wobei die flüssige Fraktion (H4) der Kontaktvorrichtung 5 zugeführt wird, um neues Substrat zu durchtränken.

12. Verfahren nach einem der Ansprüche 1 bis 11, wobei in den Reaktoren (1) und (2) die Betriebsbedingungen hinsichtlich der Temperatur und/oder des pH-Werts unterschiedlich sind.

13. Vorrichtung zur Herstellung von zuckerhaltigem Saft aus Lignocellulose-Substrat durch enzymatische Hydrolyse, aufweisend:
- mindestens einen ersten Reaktor (1) zur enzymatischen Hydrolyse der Lignocellulose, wobei der Reaktor (1) mit mindestens einer Leitung, die dazu dient, Substrat und Cellulasenlösung zuzuführen, mit mindestens einer Leitung, die dazu dient, den umgewandelten Saft H1 abzuziehen und ihn mindestens einem Filtermedium (3) zuzuführen, welches an der Austrittsöffnung des Reaktors (1) angeordnet ist, mit mindestens einem Trennmittel (7), das dazu dient, die Cellulasen aus dem Hydrolysat zu entfernen, versehen ist.
- möglicherweise einen Wärmetauscher an der Austriftsöffnung des ersten Reaktors,
- mindestens einen zweiten Reaktor (2), der die geträgerten β-Glucosidasen umfasst und über mindestens eine Leitung mit mindestens einem Trennmittel (7) verbunden ist, wobei der Reaktor (2) an der Austrittsöffnung mit mindestens einem Filtermedium (4) und mindestens einer Leitung zur Entnahme des Stoffstroms H2, welcher aus dem Hydrolysat besteht, das arm an löslichen Oligomeren ist, ausgestattet ist,
- mindestens eine Leitung, die dazu dient, mindestens einen Teil des Stoffstroms H2 in eine Kontaktvorrichtung (5) einzuleiten, und mindestens eine Leitung, die dazu dient, mindestens einen Teil des Stoffstroms H2 in den Reaktor (1) zurückzuführen,
- mindestens eine Kontaktvorrichtung (5), die mit mindestens einer Leitung, die dazu dient, neues Substrat einzuführen, mit mindestens einer Leitung, die dazu dient, den hergestellten zuckerhaltigen Saft (H3) zu entnehmen, und mit mindestens einer Leitung, die dazu dient, neues, mit Cellulasen durchtränktes Substrat zu entfernen und dieses in den Reaktor (1) einzuführen, versehen ist.

14. Vorrichtung nach Anspruch 13, **dadurch gekennzeichnet, dass** sie mindestens ein Filtermedium (6), das in der Phase des vollständigen Leerens des Reaktors (1) verwendet wird, mindestens eine Leitung zur Entnahme des feststofflichen Rückstands R, mindestens eine Leitung zur Entnahme des Stoffstroms H4 aus hergestelltem zuckerhaltigem Saft und möglicherweise mindestens eine Leitung zum Einführen des Stoffstroms H4 in den Reaktor (2) enthält.

15. Vorrichtung nach einem der Ansprüche 13 oder 14, wobei es sich bei dem Reaktor (2), der die β-Glucosidasen umfasst, um einen Reaktor mit Wirbelschicht oder vom Typ gerührte Aufschlämmung handelt, oder um einen Reaktor mit Festbett.

16. Vorrichtung nach Anspruch 15, wobei es sich bei dem Filtermedium (4) um ein Gitter oder ein Schutzbett, das im Reaktor (2) angeordnet ist, handelt.
